# EUROPEAN PATENT SPECIFICATION

(11) **EP 0 871 511 B1**
(45) Date of publication and mention of the grant of the patent: **31.03.2004**
(21) Application number: 95943831.8
(22) Date of filing: 14.12.1995
(51) Int. Cl.: A61M 29/00

(54) **MEANS FOR PERFORMING ANGIOPLASTY**
SYSTEM ZUR DURCHFÜHRUNG EINER ANGIOPLASTIK
SYSTEME PERMETTANT D'EFFECTUER UNE ANGIOPLASTIE

(43) Date of publication of application: 21.10.1998
(73) Proprietor: Pierpont Family Limited Partnership, St. Petersburg, FL 33716 (US)
(72) Inventor: Pierpont, Brien E., St. Petersburg, FL 33704 (US)
(74) Representative: Thomson, Paul Anthony
(86) International application number: PCT/US1995/016375
(87) International publication number: WO 1997/021460

(56) References cited:
- US-A- 4 911 163
- US-A- 5 158 540
- US-A- 5 178 608
- US-A- 5 304 132
- US-A- 5 484 412

## Description

Cardiac catheterization and angioplasty are common medical procedures. The coronary arteries are vessels which supply the heart muscle with blood and are located on the outside surface of the heart. In order to visually examine the coronary arteries, a contrast agent has to be injected into the vessels before x-ray pictures can be taken of them. This is accomplished through a procedure called cardiac catheterization. This contrast agent is delivered through a catheter, which is a small hollow tube. This catheter is advanced to the heart under x-ray guidance, usually being inserted at the level of the groin into the femoral artery. This is accomplished through a needle which is first advanced into the femoral artery and subsequently the catheter is passed through the needle into the blood vessel or femoral artery. The femoral artery in turn is a tributary of the great vessel originating in the heart and therefore the catheter can be passed in a retrograde fashion under x-ray guidance very easily back to the origin of the coronary arteries.

Once the catheter is positioned at the origin of the coronary arteries, a dye syringe is placed on the end of the catheter remaining outside the patient and injections are performed with simultaneous x-ray pictures being taken.

An angioplasty procedure is similar in technique but more invasive, by the fact that a smaller catheter with a deflated balloon on its tip is advanced through the catheter which is positioned at the origin of the coronary artery and advanced down into the coronary artery to the site of where the vessel is narrowed. The balloon dilatation catheter is not advanced down the coronary artery by itself, however, first, a very small guide wire is advanced down the coronary artery, across the narrowed segment and then advanced further down into the coronary artery, beyond the narrowed segment. The balloon dilatation catheter is then advanced over the guide wire to the site of the narrowing. The guide wire allows the balloon dilatation catheter to track over it, thereby facilitating advancement of the balloon dilatation catheter down the vessel and thus preventing damage to the vessel wall. Once the balloon is positioned at the site of the narrowing in the vessel, the balloon is inflated by means of a hand held balloon inflation device. The balloon is inflated for generally two to three minutes and then deflated and withdrawn. This compresses the fatty-like material which is responsible for narrowing the coronary artery and opens the vessel, allowing for proper blood flow to the heart muscle.

At times it is difficult to advance the balloon dilatation catheter to the site of the narrowing, as these vessels are not always straight and often times bends in the vessel have to be negotiated to approach the point of narrowing. Frequently, the balloon dilatation catheter cannot be easily advanced and the guiding catheter which is housing the balloon dilatation catheter and the guide wire, comes loose from its position at the origin of the coronary artery and does not provide enough structural support or backup to allow advancement of the balloon dilatation catheter to the narrowed site.

The purpose of my invention is to secure the guiding catheter to its position in the origin of the coronary artery, (coronary ostium), so that the balloon dilatation catheter can be housed therein and advanced forwardly into the coronary artery without losing structural support or backup from the guiding catheter. When the guiding catheter remains in a fixed position, it facilitates immensely the ability to advance the balloon dilatation catheter. At times angioplasties are unsuccessful purely on the basis of not being able to find a guiding catheter which will allow enough structural support to advance the balloon dilatation catheter properly.

Various balloon catheters have been developed for use in medical situations. US Patent No. 5,178,608 discloses a catheter with expandable inflation members, one of which can be inflated to provide the desired therapy. US Patent No. 5,304,132 also discloses a catheter with two balloons, both of which are used in the actual therapy, that have different lengths and diameters when inflated.

The catheter assembly disclosed in US Patent No. 5,158,540 comprises two balloons, the first to apply the therapy and the second to seal the gap between the guide catheter and the angioplasty catheter.

US Patent No. 4,911,163 teaches of a catheter with two balloons that are inflated to enclose an amount of fluid from within a vessel, whilst in situ, so as to permit diagnostic tests.

None of the above documents adequately address the problem of the present invention.

It is therefore a principal object of this invention to provide means to secure or anchor the guiding catheter in the origin of the coronary artery so that the balloon dilatation can be easily advanced therefrom into the coronary artery without losing its structural support from the guiding catheter.

### SUMMARY OF THE INVENTION

This invention comprises a catheter assembly according to claim 1. Internal balloons in the anchoring catheter can be inflated to anchor it to the balloon dilatation catheter. External balloons on the anchoring catheter can be inflated to anchor it to the inside of the guiding catheter. The external fixation balloons can be selectively inflated and deflated from the internal fixation balloons. Other external balloons on the distal end and outer surface can be inflated to secure the anchoring catheter within the blood vessel beyond the distal end of the guiding catheter. Perforations appear in the anchor catheter to permit blood to flow inside and outside thereof.

The method of using the catheter assembly, consists in inserting a conventional guide catheter into the origin of the coronary artery, (coronary ostium). The conventional guide wire, conventional balloon dilatation catheter, and anchoring catheter are then inserted through the guiding catheter with the anchoring catheter and balloon dilatation catheter being secured together by the internal balloons on the anchor catheter. As the balloon dilatation catheter and anchoring catheter are advanced over the guide wire and into the coronary artery, the internal fixation balloons are deflated and the spaced external fixation balloons are inflated, thus securing the anchoring catheter to both the guiding catheter and the interior of the proximal portion of the coronary artery. This therefore serves to secure the guiding catheter to the origin of the coronary artery. The balloon dilatation catheter is then extended from the anchoring catheter tracking over the guide wire to perform the conventional function with respect to the narrowed section of the blood vessel. The method of using the catheter is not part of the invention.

### BRIEF DESCRIPTION OF THE DRAWINGS

Fig. 1 is a schematic view of a human heart with the apparatus of this invention inserted into a coronary artery;
Fig. 2 is an enlarged scale schematic view of the segment A in Fig. 1;
Fig. 3 is a large scale sectional-perspective view of the forward or inner ends of the balloon dilatation, anchor and guiding catheters;
Fig. 4 is a typical cross-sectional view at an enlarged scale taken on line 4-4 of Fig. 3,
Fig. 5 is an enlarged scale sectional view of Fig. 2;
Fig. 6 is an end elevational view of an inflation valve for the internal and external balloons of the anchoring catheter;
Fig. 7 is a schematic sectional view through the valve of Fig. 6 when the internal balloons are inflated; and
Fig. 8 is a schematic sectional view through the valve of Fig. 6 when the external balloons of the anchoring catheter are inflated.

### DESCRIPTION OF THE PREFERRED EMBODIMENT

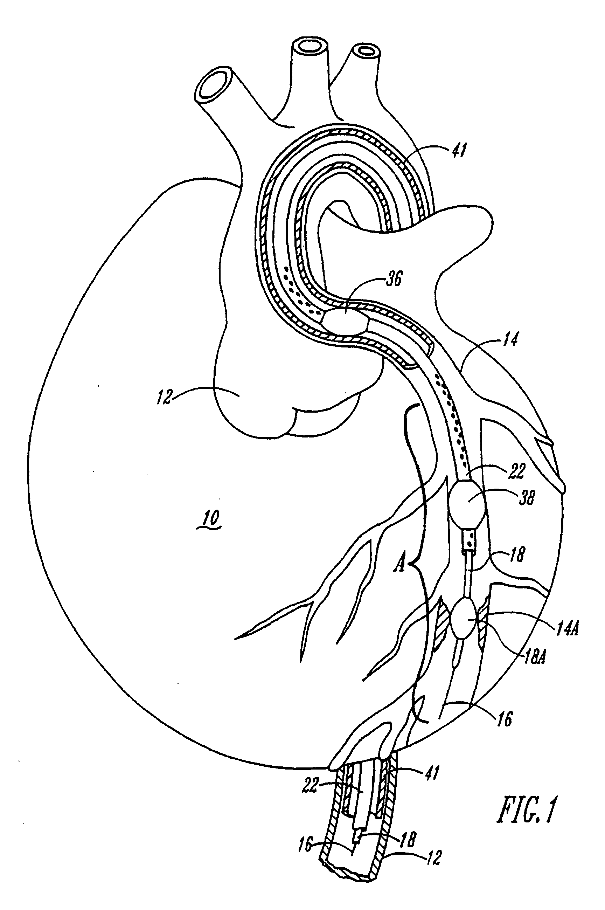

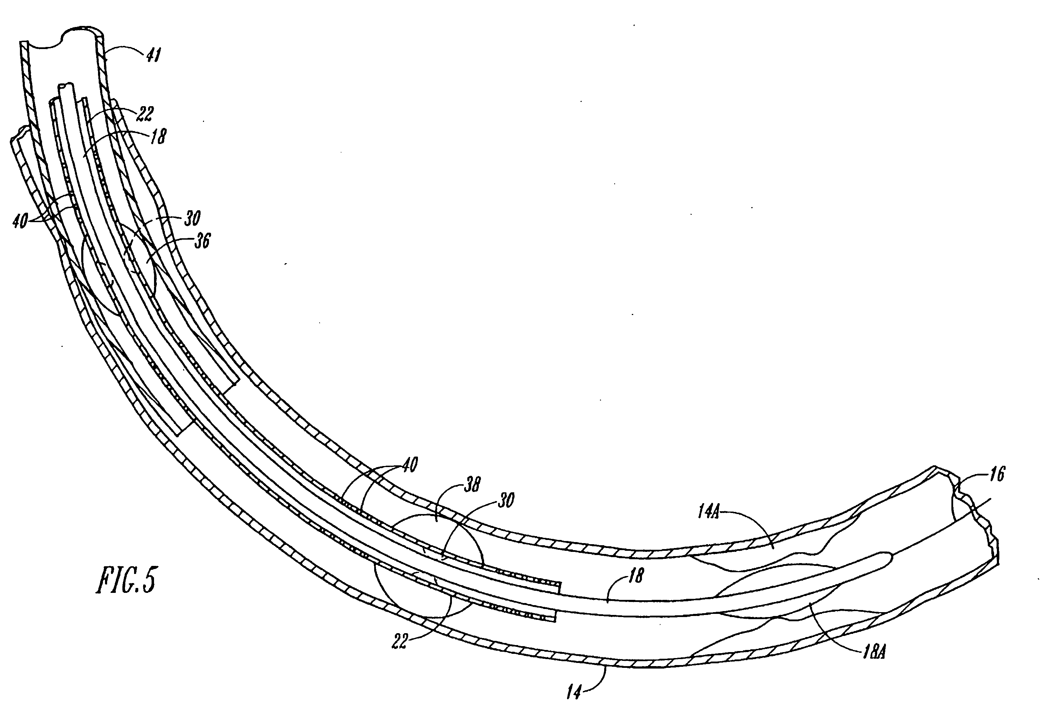

Figure 1 shows a schematic view of a heart muscle 10 connected to the primary blood supply vessel 12 (aorta). A coronary artery 14 is also depicted in Fig. 1. The numeral 14A in Fig. 1 and Fig. 5 shows the plaque or obstruction in the coronary artery 14.

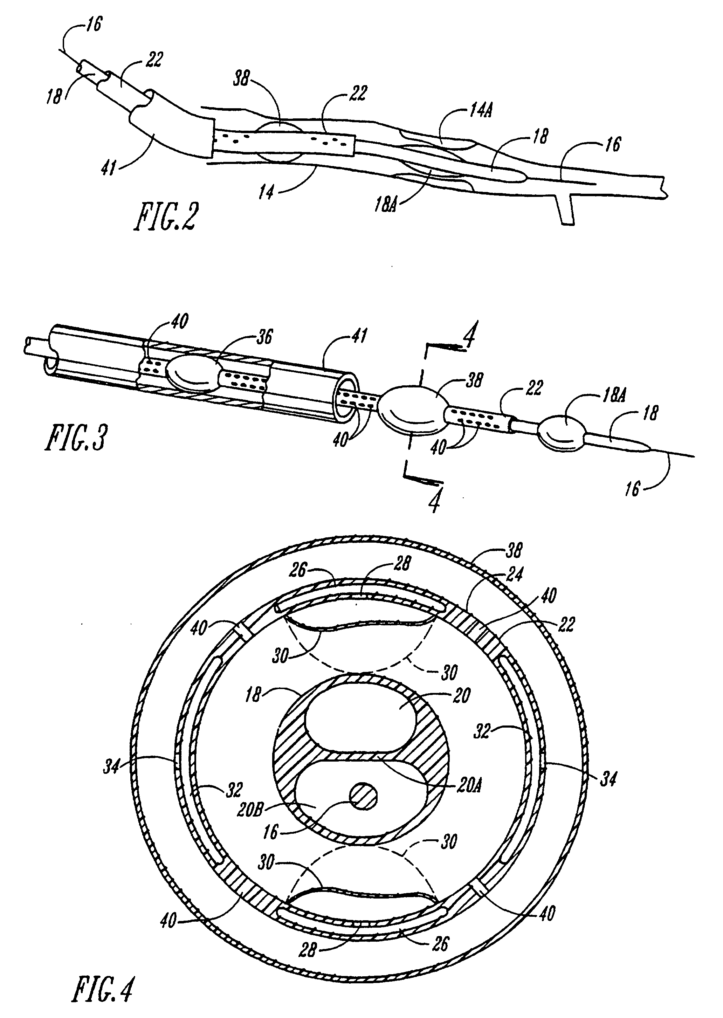

With reference to Fig. 5, the numeral 16 reveals a conventional guide wire 16 upon which a balloon dilatation catheter 18 is slidably mounted. Catheter 18 has an inflatable balloon 18A on the inner end thereof. Catheter 18 has its internal diameter divided by membrane 20A to create a balloon inflation passageway 20 and a guide wire passageway 20B (Fig. 4).

Balloon dilatation catheter 18 is slidably mounted within the hollow interior of anchor catheter 22. The outer wall 24 of anchor catheter 22 has two hollow elongated external balloon inflation passageways 26, each of which has a port 28. Two pairs of flexible internal balloons 30 extend inwardly within the hollow interior of anchor catheter 22 and extend over ports 28.

Similarly, the outer wall 24 of anchor catheter 22 has two external balloon passageways 32 (Fig. 4) each of which have external ports 34. A first concentric external balloon 36 extends outwardly from wall 24 over a first pair of ports 34, and a second concentric external balloon 38 extends over a second pair of ports 34. As also seen in Fig. 4, a plurality of blood profusion ports 40 extend through wall 24 of anchor catheter 22 on opposite sides of balloons 36 and 38.

The numeral 41 (Fig. 5) designates a guiding catheter whose function will be described hereafter.

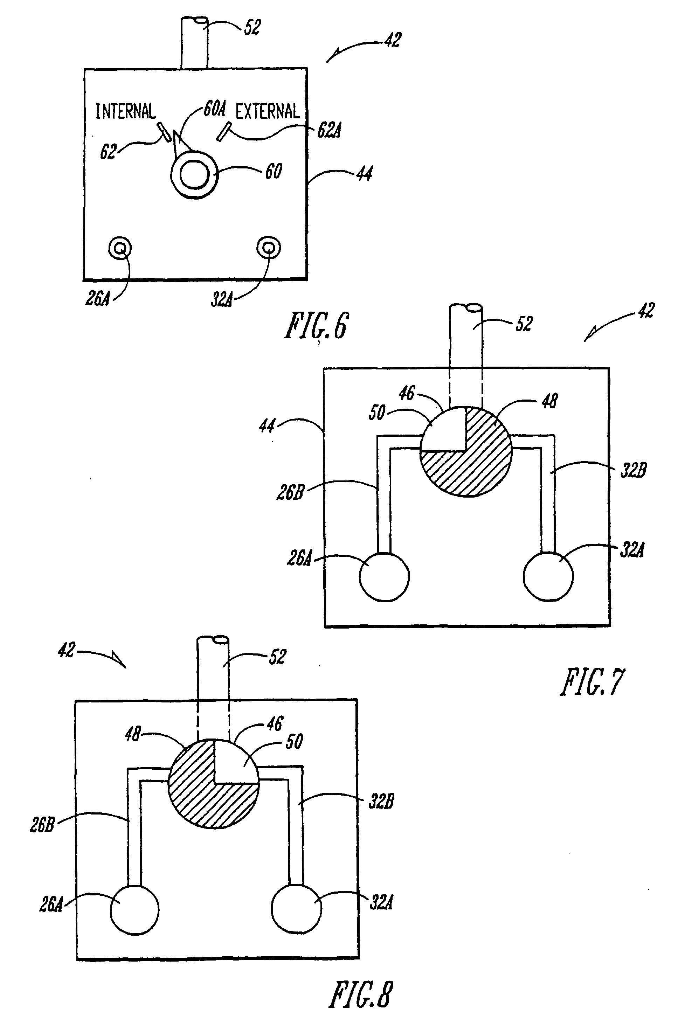

Figure 6 shows an end elevation of a balloon inflation valve 42 which has a body 44 depicted in Fig. 7. A horizontal bore 46 extends into body 44 and rotatably supports spool 48 which has an elongated 90 degree notch 50 which is in communication with a contrast agent inlet 52 which is also in communication with bore 46.

Horizontal bores 26A and 32A also extend into body 44 and are adapted to be connected (not shown) with internal balloon inflation passageways 26 and external balloon inflation passageways 32, respectively.

A conventional knob 60 (Figure 6) is mounted on the outer end of spool 48 (Fig. 7) and has a guide tab 60A thereon which is adapted to engage stop element 62 or 62A which are embossed on the outer face of body 44 as shown in Fig. 6. When tab 60A engages stop element 62, the spool 48 is in the position of Fig. 7 to introduce contrast agent through passageway 52, bore 46, and passageway 26B to introduce air under pressure into bore 26A whereupon the internal balloon inflation passageways 26 will receive a source of contrast agent to inflate the flexible internal balloons 30 of anchoring catheter 22. When the spool 48 is changed from the position of Fig. 7, as just described, to the position of Fig. 8, contrast agent is introduced in a similar manner but through passageway 32A to cause the two external balloons 36 (depicted in Fig. 3) and 38 to inflate in like manner. It should be noted that when the valve spool 46 is moved from the position of Fig. 7 to Fig. 8, or vice versa, only one of the passageways 26A or 32A will have contrast agent, while contrast agent will be denied to the other of such passageways. The spool will allow only the internal balloons or the external balloons to inflate, but not both at the same time. Thus, the valve 42 will guarantee that the internal and external balloons of the anchoring catheter 22 will not both be inflated simultaneously. This prevents any injury to the patient by preventing any shearing force which could be created if both sets of balloons were inflated simultaneously and an attempt to advance the balloon dilatation catheter was made resulting in dragging the anchoring catheter and externally inflated balloons along with it.

In operation, the guiding catheter 41 is inserted into the groin of the patient in the manner described above and positioned at the origin of the coronary artery 14. The balloon dilatation catheter 18 is inserted over guide wire 16, and both the guide wire 16, and both the guide wire 16 and balloon catheter 18 are then inserted within anchoring catheter 22. The assembled guide wire 16, balloon dilatation catheter 18 and anchoring catheter 22 are moved as a unit into guiding catheter 41 after the internal fixation balloons 30 of the anchoring catheter have been inflated in the manner described. At this point in time, the external balloons 36 and 38 of anchoring catheter 22 are still deflated. The above described assembled components are extended through the guiding catheter until the guide wire 16 and the distal end of the anchoring catheter 22 extend distally beyond the distal end of the guiding catheter. At that point in time, the internal balloons 30 are deflated in the manner described by valve 42 and the external balloons 36 and 38 are inflated. The external balloon 38 engages the inner wall of coronary artery 14, while the external balloon 36 engages the interior of the guiding catheter 41. This secures the guiding catheter 41 to the coronary artery 14.

With deflation of the internal fixation balloons, the balloon dilatation catheter 18 is now movable independent of the anchoring catheter and is thereupon moved to the position shown in Figures 1, 2 and 5 so that the balloon 18A is adjacent the plaque 14A. This can be easily accomplished by the reason of the fact that the anchoring catheter is secured both to the internal wall of the blood vessel and to the internal surface of the guiding catheter 41, anchoring the guiding catheter to the coronary ostium, thus providing backup support for advancement of the balloon dilatation catheter over the guide wire. The balloon 18A is then inflated in the conventional manner to treat the plaque 14A. After this has been done, the external balloons 36 and 38 are deflated, and the respective catheters can be removed in the conventional fashion.

The perfusion ports 40 allow for blood to flow through the anchoring catheter and down the coronary artery while the balloons are inflated. This is very critical so as to continue to support the heart muscle 10 with oxygen while the balloons are inflated and anchoring the guiding catheter to the coronary artery.

It is therefore seen that this invention will accomplish at least of its stated objectives.

## Claims

1. A catheter assembly, **characterised by**
an elongated hollow anchoring catheter (22) having a distal end, and a tubular wall (24) with inner and outer surfaces,
a hollow guiding catheter (41) having a distal end and a proximal end housing said anchoring catheter (22)
a first anchoring balloon member (36) attached to the outer surface of said tubular wall (24) of the anchoring catheter and adapted upon inflation to project outwardly from said tubular wall to engage the guiding catheter (41) and secure said anchoring catheter within said guiding catheter,
an elongated balloon dilatation catheter (18) longitudinally extending through said anchoring catheter (22) and having a distal end,
a dilatation balloon (18A) attached to the distal end of said balloon dilatation catheter,
a second anchoring balloon member (30) attached to the inner surface of said tubular wall (24) and adapted upon inflation to project inwardly from said tubular wall of the anchoring catheter to engage and retain said dilatation catheter (18) against movement with respect to said anchoring catheter (22),
means associated with said catheter assembly for independently inflating and deflating said first and second anchoring balloon members;
means for inflating the dilatation balloon; and
a guide wire (16) extending through the dilatation catheter (18) and along which the dilatation catheter is slidable.

2. The assembly of claim 1 further comprising a third anchoring balloon member (38) attached to the outer surface of the tubular wall of the anchoring catheter (22) and adapted upon inflation to project outwardly to engage the blood vessel (14) and secure the anchoring catheter (22) to the blood vessel, and whereby upon inflation of the first (36) and third (38) balloon members the guiding catheter (41) is operatively secured to the blood vessel.

3. The catheter assembly of claim 1 further comprising first and second perfusion ports (40) in the anchoring catheter (22) on opposite sides of the third anchoring balloon member (38) to allow blood flow through the anchoring catheter while the third anchoring balloon member is inflated.

4. The assembly of claim 1 wherein a longitudinally spaced pair of said second anchoring balloon members (30) are attached to said tubular wall (24) of the anchoring catheter (22).

5. The assembly of claim 1 wherein a pair of each of said first and second balloon members (36, 30) are attached to said tubular wall (24) of the anchoring catheter(22).

6. The assembly of claim 1 wherein blood by-pass means are located in said tubular wall (24) on opposite sides of at least one of said first or second anchoring balloon members(36, 30).

7. The assembly of claim 1 wherein a longitudinally spaced pair of said first anchoring balloon members (36) are attached to said tubular wall (24) of the anchoring catheter(22).

8. The catheter assembly of claim 7 wherein one of the pairs of first anchoring balloon members (36) secures aid anchoring catheter (22) to the blood vessel(14).

9. The catheter assembly of claim 1 further comprising first and second perfusion ports (40) in the anchoring catheter (22)on opposite sides of the first anchoring balloon (36) to allow blood flow through the anchoring catheter (22) while the first anchoring balloon (36) is inflated.

## Patentansprüche

1. Katheteranordnung **gekennzeichnet durch**
einen länglichen hohlen Verankerungskatheter (22) mit einem distalen Ende und einer schlauch- oder röhrenförmigen Wand (24) mit Innen- und Außenflächen,
einen hohlen Führungskatheter (41) mit einem distalen Ende und einem proximalen Ende, der den Verankerungskatheter (22) aufnimmt,
ein erstes Verankerungsballonelement (36), welches an der Außenfläche der schlauch- oder röhrenförmigen Wand (24) des Verankerungskatheters angebracht und derart ausgebildet ist, dass es im aufgeblasenen Zustand von der schlauch- oder röhrenförmigen Wand nach außen absteht und mit dem Führungskatheter (41) in Eingriff gelangt und den Verankerungskatheter innerhalb des Führungskatheters fixiert,
einen länglichen Ballondilatationskatheter (18), der längs **durch** den Verankerungskatheter (22) führt und ein distales Ende aufweist,
einen Dilatationsballon (18A), der am distalen Ende des Ballondilatationskatheters angebracht ist,
ein zweites Verankerungsballonelement (30), das an der Innenfläche der schlauch- oder röhrenförmigen Wand (24) angebracht und derart ausgebildet ist, dass es im aufgeblasenen Zustand von der schlauch- oder röhrenförmigen Wand des Verankerungskatheters nach innen absteht und mit dem Dilatationskatheter (18) in Eingriff gelangt und diesen daran hindert, sich relativ zum Verankerungskatheter (22) zu bewegen,
eine zur Katheteranordnung gehörende Vorrichtung zum unabhängigen Aufblasen und Entleeren des ersten und des zweiten Verankerungsballonelements; eine Vorrichtung zum Aufblasen des Dilatationsballons; und
einen Führungsdraht (16), der **durch** den Dilatationskatheter (18) führt und entlang dessen der Dilatationskatheter verschiebbar ist.

2. Anordnung nach Anspruch 1, die des weiteren ein drittes Verankerungsballonelement (38) aufweist, das an der Außenfläche der schlauch- oder röhrenförmigen Wand des Verankerungskatheters (22) angebracht und derart ausgebildet ist, dass es im aufgeblasenen Zustand nach außen absteht und mit dem Blutgefäß (14) in Eingriff gelangt und den Verankerungskatheter (22) an dem Blutgefäß fixiert, wobei der Führungskatheter (41) durch Aufblasen des ersten (36) und dritten (38) Ballonelements wirksam an dem Blutgefäß fixiert ist.

3. Katheteranordnung nach Anspruch 1, des weiteren mit an sich gegenüberliegenden Seiten des dritten Verankerungsballonelements (38) im Verankerungskatheter (22) angeordneten ersten und zweiten Perfusionsports (40) zur Gewährleistung des Blutflusses durch den Verankerungskatheter während des Aufblasens des Verankerungsballonelements.

4. Anordnung nach Anspruch 1, wobei ein in Längsrichtung von einander beabstandetes Paar zweiter Verankerungsballonelemente (30) an der schlauchoder röhrenförmigen Wand (24) des Verankerungskatheters (22) angebracht ist.

5. Anordnung nach Anspruch 1, wobei jeweils ein Paar erster und zweiter Ballonelemente (36, 30) an der schlauch- oder röhrenförmigen Wand (24) des Verankerungskatheters (22) angebracht ist.

6. Anordnung nach Anspruch 1, wobei eine Blut-By-Pass-Vorrichtung an der schlauch- oder röhrenförmigen Wand (24) an sich gegenüberliegenden Seiten mindestens eines der ersten und zweiten Verankerungsballonelemente (36, 30) angebracht ist.

7. Anordnung nach Anspruch 1, wobei ein in Längsrichtung voneinander beabstandetes Paar des ersten Verankerungsballonelements (36) an der schlauchoder röhrenförmigen Wand (24) des Verankerungskatheters (22) angebracht ist.

8. Katheteranordnung nach Anspruch 7, wobei eines der Paare erster Verankerungsballonelemente (36) den Verankerungskatheter (22) an dem Blutgefäß (14) fixiert.

9. Katheteranordnung nach Anspruch 1, des weiteren mit an sich gegenüberliegenden Seiten des ersten Verankerungsballons (36) im Verankerungskatheter (22) angeordneten ersten und zweiten Perfusionsports (40) zur Gewährleistung des Blutflusses durch den Verankerungskatheter (22) während des Aufblasens des ersten Verankerungsballons (36).

## Revendications

1. Assemblage de cathéter, **caractérisé par**
un cathéter à ancrage creux allongé (22) possédant une extrémité distale, et une paroi tubulaire (24) avec des surfaces intérieure et extérieure,
un cathéter de guidage creux (41) possédant une extrémité distale et une extrémité proximale renfermant ledit cathéter à ancrage (22)
un premier élément de ballon d'ancrage (36) relié à la surface extérieure de ladite paroi tubulaire (24) du cathéter à ancrage et adapté lors du gonflage pour se projeter vers l'extérieur de ladite paroi tubulaire afin d'engager le cathéter de guidage (41) et de fixer ledit cathéter à ancrage sur ledit cathéter de guidage,
un cathéter à dilatation allongé à ballon (18) s'étendant longitudinalement le long dudit cathéter à ancrage (22) et possédant une extrémité distale,
un ballon de dilatation (18A) relié à l'extrémité distale dudit cathéter à dilatation en forme de ballon,
un deuxième élément de ballon d'ancrage (30) relié à la surface intérieure de ladite paroi tubulaire (24) et adapté lors du gonflage pour se projeter vers l'intérieur à partir de ladite paroi tubulaire du cathéter à ancrage afin d'engager et de retenir ledit cathéter à dilatation (18) contre tout mouvement par rapport audit cathéter à ancrage (22),
un moyen associé audit assemblage de cathéter pour gonfler et dégonfler indépendamment lesdits premier et deuxième éléments de ballon ;
un moyen pour gonfler le ballon de dilatation ; et
un fil de guidage (16) s'étendant à travers le cathéter à dilatation (18) et le long duquel le cathéter à dilatation peut coulisser.

2. Assemblage selon la revendication 1, comprenant en outre un troisième élément de ballon d'ancrage (38) relié à la surface extérieure de la paroi tubulaire du cathéter à ancrage (22) et adapté lors du gonflage pour se projeter vers l'extérieur afin de s'engager dans le vaisseau sanguin (14) et de fixer le cathéter à ancrage (22) au vaisseau sanguin, et moyennant quoi, lors du gonflage des premier (36) et troisième (38) éléments de ballon, le cathéter de guidage (41) est fixé au vaisseau sanguin de manière opérationnelle.

3. Assemblage de cathéter selon la revendication 1, comprenant en outre un premier et un deuxième ports de perfusion (40) au sein du cathéter à ancrage (22) sur les côtés opposés du troisième élément de ballon d'ancrage (38) afin de permettre un écoulement sanguin dans le cathéter à ancrage pendant que le troisième élément de ballon d'ancrage est gonflé.

4. Assemblage selon la revendication 1, dans lequel une paire longitudinalement espacée desdits deuxièmes éléments d'ancrage (30) est reliée à ladite paroi tubulaire (24) du cathéter à ancrage (22).

5. Assemblage selon la revendication 1, dans lequel une paire de chacun desdits premier et deuxième éléments de ballon (36, 30) est reliée à ladite paroi tubulaire (24) du cathéter à ancrage (22).

6. Assemblage selon la revendication 1, dans lequel des moyens de dérivation sanguine sont situés dans ladite paroi tubulaire (24) sur les côtés opposés d'au moins l'un desdits premier et deuxième éléments de ballon d'ancrage (36, 30).

7. Assemblage selon la revendication 1, dans lequel une paire longitudinalement espacée desdits premiers éléments de ballon d'ancrage (36) est reliée à ladite paroi tubulaire (24) du cathéter à ancrage (22).

8. Assemblage de cathéter selon la revendication 7, dans lequel l'une des paires de premiers éléments de ballon d'ancrage (36) fixe le cathéter à ancrage (22) au vaisseau sanguin (14).

9. Assemblage de cathéter selon la revendication 1, comprenant en outre un premier et un deuxième ports de perfusion (40) au sein du cathéter à ancrage (22) sur les côtés opposés du premier ballon d'ancrage (36) afin de permettre un écoulement sanguin à travers le cathéter à ancrage (22) pendant que le premier ballon d'ancrage (36) est gonflé.
